Europäisches Patentamt

European Patent Office

Office européen des brevets

(19)

(11) **EP 1 093 786 B1**

(12) **EUROPEAN PATENT SPECIFICATION**

(45) Date of publication and mention
of the grant of the patent:
**01.03.2006 Bulletin 2006/09**

(51) Int Cl.:
*A61K 8/97* (2006.01)    *A61Q 19/08* (2006.01)

(21) Application number: **00309279.8**

(22) Date of filing: **20.10.2000**

(54) **Skin cosmetic composition containing kidney bean extracts**

Hautkosmetische Zubereitung einen Extrakt weisser Bohnen enthaltend

Composition cosmétique pour la peau comprenant un extrait d'haricots blancs

(84) Designated Contracting States:
**CH DE ES FR GB IT LI**

(30) Priority: **21.10.1999 KR 9945792**

(43) Date of publication of application:
**25.04.2001 Bulletin 2001/17**

(73) Proprietor: **Coreana Cosmetics Co., Ltd.**
**Cheonan-si,**
**Chungcheongnam-do (KR)**

(72) Inventors:
 • **Lee, Kang Tae**
 **CheonAn-si,**
 **Chungcheongnam-do 330-810 (KR)**
 • **Shin, Bong Soo**
 **Kimcheon-si,**
 **Kyongsangbuk-do 740-060 (KR)**
 • **Yoo, Young Kyoung**
 **Chungcheongpook-do (KR)**
 • **Kim, Sung Woo**
 **Jung-gu,**
 **Taejon 301-152 (KR)**

(74) Representative: **Dean, John Paul et al**
**Withers & Rogers LLP**
**Goldings House,**
**2 Hays Lane**
**London SE1 2HW (GB)**

(56) References cited:
 **FR-A- 2 759 295**       **US-A- 4 383 833**

 • **DATABASE WPI Section Ch, Week 199223
 Derwent Publications Ltd., London, GB; Class
 D21, AN 1992-189208 XP002160629 & JP 04
 124124 A (MORISHITA JINTAN KK), 24 April 1992
 (1992-04-24)**
 • **DATABASE WPI Section Ch, Week 198429
 Derwent Publications Ltd., London, GB; Class
 D21, AN 1984-180134 XP002160630 & JP 59
 101414 A (SUNSTAR KK), 12 June 1984
 (1984-06-12)**
 • **PATENT ABSTRACTS OF JAPAN vol. 1997, no.
 05, 30 May 1997 (1997-05-30) & JP 09 025225 A
 (SANSHO SEIYAKU CO LTD), 28 January 1997
 (1997-01-28)**
 • **PATENT ABSTRACTS OF JAPAN vol. 018, no. 400
 (C-1230), 26 July 1994 (1994-07-26) & JP 06 116197
 A (MAX FUAKUTAA KK), 26 April 1994
 (1994-04-26)**
 • **PATENT ABSTRACTS OF JAPAN vol. 012, no. 297
 (C-519), 12 August 1988 (1988-08-12) & JP 63
 066109 A (KANEBO LTD), 24 March 1988
 (1988-03-24)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

### Field of the Invention

The present invention is related to a skin cosmetic composition containing kidney bean extracts.

### Background of the Invention

In cosmetics-related field, the most critical task in preventing skin ageing is suppression of wrinkle formation or improvement of wrinkle. Female hormone, estrogen stimulates fibroblast in dermis to accelerate collagen synthesis and metabolism, as well as to stimulate synthesis of hyaluronic acid, which is very important for skin moisture retention. In addition, estrogen helps proliferation of basal layer cell in epidermis, which makes epidermis thicker to reinforce skin cell tissue. Further, estrogen reduces the formation of sebum by inhibiting sebaceous gland growth through control of sebaceous gland, and keeps hair thin and short by reduction of growth rate thereof. In particular, estrogen is involved in collagen metabolism, inhibits over-glycation of collagen, induces post-translational modification, and inhibits degradation of collagen by regulating the expression of collagenase, the degradation enzyme of collagen. As such, major function of estrogen on skin is to reinforce skin action and to keep skin smooth, elastic and soft.

However, as one grows older, estrogen-secreting function of the genital organ declines, and around menopause period when the production function is lost, the formation of estrogen is stopped. When hormone balance is lost by the stop of estrogen formation, because of the non-competitive effect of male hormone, testosterone, endocrine ageing with estrogen deficiency is stimulated. The endocrine ageing is a type of physiological skin ageing, which occurs at menopause period.

Estrogen deficiency causes skin changes, and a typical phenomenon is skin drying. Because of estrogen deficiency, formation of collagen and elastic fibre is suppressed in fibroblast, which leads to thinner skin and reduction of skin elasticity. Additionally, reduction in synthesis of hyaluronic acid results in weakening of moisture retention function of skin. Further, cross-linking of collagen increases due to increase in collagen glycation, resulting in skin hardening and elasticity reduction.

Studies on a method for inhibiting skin ageing due to estrogen deficiency have been reported. Among these, hormone replacement therapy involves direct supply of estrogen. The direct supply of estrogen brought remarkable effects such as increase in collagen synthesis, hyaluronic acid synthesis and epidermis cell proliferation leading to an increase in epidermis thickness.

However, as it is not allowed to use estrogen itself in cosmetics, alternative methods have been sought. In cosmetic field, instead of direct method for estrogen deficiency, indirect method using general inhibitory substance against skin ageing such as moisturising substance, anti-ageing substance, free radical eliminating substance or UV blocking agent.

In JP04124124A, a buccal composition containing a kidney bean extract is disclosed as being useful for deodorising bad breath. A similar extract is described in JP59101414A for use as a hair treatment. JP09025225A describes extracts of a wide range of beans, one of which is kidney bean, for the manufacture of a skin treatment for improving chapping. A range of compounds extracted from kidney beans is disclosed in JP06116197A as being useful as antioxidants. In JP63066109A, skin cosmetic compositions containing 2-dodecenedioic acid obtained from pods of leguminous plants, such as kidney beans, are used for improving the water retaining function and gloss of the skin.

### Summary of the Invention

To improve female skin where ageing is rapidly progressed around menopause period, it is important to develop a substance having function similar to estrogen. And as for the estrogen-like substance, the functions of collagen synthesis and hyaluronic acid synthesis are required, from the viewpoint of cosmetics to inhibit skin ageing.

The inventors of the present invention have studied on wild herb medicine to develop estrogen-like substance, and found that kidney bean extract shows a superior effect on cell proliferation, collagen synthesis and hyaluronic acid synthesis, and completed the present invention.

### Detailed Description of the Invention

The present invention provides the use of a kidney bean extract in the preparation of a skin cosmetic composition for improving skin wrinkles.

In the cosmetic composition of the present invention, kidney bean extracts is preferably contained in an amount of 0.05 to 10.0% by weight based on dried weight of the cosmetic composition.

Kidney bean belongs to Fabaceae family, and includes vine kidney bean (Phaseolus vulgaris L.), red kidney bean (Phaseolus multiflorus WILD), kidney bean (Phaseolus vulgaris L. var. humilis ALEF) and white kidney bean (Phaseolus multiflorus WILD for albus BAIKEY), and these all can be used in the present invention.

The kidney bean extracts according to the present invention are prepared as follows. Firstly, kidney bean seeds are dried, washed with purified water, dried and pulverised. As extraction solvent, water, lower alcohol (e.g. methanol, ethanol), mixture of water and lower alcohol, acetone, ethyl acetate, 1,3-butylene glycol, hexane, diethyl ether, n-propanol, isopropanol, or n-butanol is added to 1 to 15 times of dried weight of the pulverised kidney bean, and deposited for 1 to 15 days at an ambient temperature to extract active ingredient. Thus extracted solution was subjected to vacuum concentration in a distillation apparatus with cooling condenser to form kidney bean extracts.

The kidney bean extracts of the present invention may be contained in various cosmetics such as basal cosmetics (e.g., softener, cream, essence, cleansing foam, cleansing water, pack and body oil), colour cosmetics (e.g., foundation, lipstick, mascara and make up base), and hair cosmetics (e.g., shampoo, hair conditioner and hair gel) can be enumerated.

In order that this invention may be better understood, the following examples are set forth. These examples are for purposes of illustration only and are not to be construed as limiting the scope of this invention in any manner.

### Example 1

Kidney bean was washed with purified water, dried and pulverised to obtain kidney bean powder. 1 kg of kidney bean powder was then mixed with 5 L of water, and extracted for 5 days at ambient temperature. The extracts were filtered with 300-mesh filter cloth, and were left for 10 days at the temperature of 4-15°C (i.e. low temperature maturation), and then filtered with Whatman No. 2 filter paper. These extracts were subjected to vacuum concentration at 70°C in a distillation apparatus with condenser, and dried to obtain 132.20 g (dried weight) of kidney bean extracts. Extraction yield was 13.2 ±0.8%.

### Example 2

1 kg of kidney bean powder which was obtained by washing kidney bean with purified water, drying and pulverisation, was mixed with 5 L of 10% ethanol, and then extracted for 3 days in extractor with condenser. The extracts were filtered with 300-mesh filter cloth, and matured for 10 days at 4 to 15°C, and then filtered with Whatman No. 2 filter paper. These extracts were subjected to vacuum concentration at 70°C in a distillation apparatus with condenser, and dried to obtain 127.4g (dried weight) of kidney bean extracts. Extraction yield was 127 ±0.7%.

### Examples 3 to 21

In each example, extraction was performed according to Example 2, except for using the solvent in Table 1. Each extraction yield was shown in Table 1.

| Example | Solvent | Extraction Yield (0% average ± standard deviation) |
|---|---|---|
| Example 3 | 20% ethanol | 12.1 ± 0.7 |
| Example 4 | 30% ethanol | 112 ± 0.6 |
| Example 5 | 40% ethanol | 9.9 ± 0.4 |
| Example 6 | 50% ethanol | 8.4 ±1.0 |
| Example 7 | 60% ethanol | 6.7 ± 0.5 |
| Example 8 | 70% ethanol | 5.4 ± 0.5 |
| Example 9 | 80% ethanol | 4.3 ± 0.3 |
| Example 10 | 90% ethanol | 2.5 ± 0.1 |
| Example 11 | 100% ethanol | 1.5 ± 0.1 |
| Example 12 | 80% methanol | 4.2 ± 0.4 |
| Example 13 | 100 methanol | 1.5 ± 0.1 |
| Example 14 | Acetone | 0.7 ± 0.1 |
| Example 15 | Ethyl acetate | 1.1 ± 0.2 |

Table continued

| Example | Solvent | Extraction Yield (0% average ± standard deviation) |
|---------|---------|------------------------------------------------------|
| Example 16 | 50% 1,3-butylene glycol aqueous solution | 5.8 ± 0.5 |
| Example 17 | Hexane | 0.8 ± 0.1 |
| Example 18 | Diethyl ether | 0.8 ± 0.1 |
| Example 19 | n-propanol | 0.2 ± 0.1 |
| Example 20 | Iso-propanol | 0.2 ± 0.1 |
| Example 21 | n-butanol | 0.2 ± 0.1 |

**Experimental Example 1**

Cell proliferation effect of kidney bean extracts

**1) Experimental method**

Human normal fibroblast was inoculated in each well of 96-well microplate ($1 \times 10^4$ cell/well) and cultivated in DMEM medium for 24 hours. After the culture, culture medium was changed to serum-free DMEM medium containing 250 μg/ml of kidney bean extracts prepared in Examples 1 to 21, and cultivated further for 24 hours. MTT solution [3-(4,5-dimethylthiazol-2-yl) 2,5-diphenyltetrazolium bromide: 5 mg/ml] 10 μl was added, and after 4 hours, medium was removed. 100 μl of dimethyl sulfoxide solution was added to each well and stirred for 20 minutes. Absorbance was measured at 570 nm using microplate reader. Same procedure was repeated for three times.

**2) Experimental result**

Cell proliferation effect was calculated by following equation and its result was shown in Table 2.

$$\text{Cell proliferation effect (\%)} = \{(\text{Absorbance of extracts} - \text{Absorbance of control})/(\text{Absorbance of control})\} \times 100$$

**Table 2**

| Example No. | Cell proliferation effect (% by weight) |
|-------------|------------------------------------------|
| Example 1 | 15.4 ± 1.3 |
| Example 2 | 18.6 ± 1.6 |
| Example 3 | 21.5 ± 2.5 |
| Example 4 | 27.6 ± 0.9 |
| Example 5 | 35.9 ± 1.3 |
| Example 6 | 42.7 ± 2.1 |
| Example 7 | 51.3 ± 2.4 |
| Example 8 | 58.7 ± 2.5 |
| Example 9 | 54.6 ± 2.5 |
| Example 10 | 56.5 ± 2.3 |
| Example 11 | 52.8 ± 2.0 |
| Example 12 | 54.5 ± 2.2 |

EP 1 093 786 B1

Table continued

| Example No. | Cell proliferation effect (% by weight) |
|---|---|
| Example 13 | $45.7 \pm 1.6$ |
| Example 14 | $31.4 \pm 1.5$ |
| Example 15 | $44.8 \pm 1.8$ |
| Example 16 | $52.1 \pm 2.4$ |
| Example 17 | $10.3 \pm 0.6$ |
| Example 18 | $11.4 \pm 0.8$ |
| Example 19 | $22.4 \pm 1.4$ |
| Example 20 | $25.3 \pm 1.6$ |
| Example 21 | $21.7 \pm 1.4$ |
| * The above values are average of three times experiments. | |

The above result clearly shows that kidney bean extracts of the present invention exhibits superior effect on cell proliferation.

**Experimental example 2**

Effect of kidney bean extracts on collagen synthesis

**1) Experimental method**

Human normal fibroblast was inoculated to 96-well microplate ($2 \times 10^4$ cell/well) and cultivated for 24 hours. After the culture, culture medium was changed to serum-free DMEM medium containing kidney bean extracts in the following Table 3, and cultivated for 48 hours. In Table 3, control group was cultivated without kidney bean extracts. Kidney bean extracts prepared in Example 8 was used. Before 24 hours from the end of culture, ascorbic acid (50 $\mu$g/ml) was added to stimulate collagen synthesis. After the culture, each well was washed and changed to serum-free DMEM medium and cultivated for another 24 hours. Supernatants of each well was collected and amount of procollagen type IC-peptide (PICP) was measured by using kit (Takara, Kyoto, Japan) and the amount of PICP was converted into ng/$2 \times 10^4$cell.

**2) Experimental result**

Kidney bean extracts of the present invention exhibited effect on synthesis of collagen I in human normal fibroblast and the result was given in Table 3.

**Table 3**

| Concentration | Amount of collagen synthesis (ng/$2 \times 10^4$ cell) |
|---|---|
| Control | 151 |
| 100 $\mu$g/ml | 204 |
| 250 $\mu$g/ml | 254 |
| 500 $\mu$g/ml | 291 |

According to the above result, synthesis yield of collagen increases as the concentration of kidney bean extracts increases, showing that kidney bean extracts according to the present invention exhibits superior effect of increasing collagen synthesis.

**Experimental example 3**

Effects of kidney bean extracts on cell proliferation

Experiment was carried out according to the procedure of Experimental example 1. Kidney bean extracts prepared in Example 8 was used in different concentration. The result is as described in Table 4.

**Table 4**

| Concentration | Absorbance (570 nm) | Cell Proliferation effect (%) |
|---|---|---|
| Control | 0.320 ± 0.008 | - |
| 100 μg/ml | 0.414 ± 0.012 | 29.4 |
| 250 μg/ml | 0.454 ± 0.030 | 41.6 |
| 500 μg/ml | 0.563 ± 0.015 | 75.9 |

The above result shows that cell proliferation effect increases as the concentration of kidney bean extracts increases, and based on this, it can be seen that kidney bean extracts of the present invention exhibits superior effect of cell proliferation.

**Experimental example 4**

Effect of kidney bean extracts on hyaluronic acid synthesis

Human normal fibroblast was inoculated to 6-well microplate ($3\times10^4$ cell/well) and cultivated for 48 hours. At this time, as culture medium, DMEM medium containing 10% fetal calf serum, penicillin and streptomycin (100 μg/ml) was used. After the culture, medium was changed to a culture medium containing various concentrations of kidney bean extracts as in Table 3 and cultivated for 24 hours. Kidney bean extracts prepared in Example 8 was used. After the culture, only medium part was collected, and then trichloroacetic acid was added to final concentration of 10% and stored at 4° C for 24 hours in order to remove protein. This solution was centrifuged, its supernatant was subjected to dialysis, it was treated with NaCl to a final concentration of 0.04 M, and then cetylpyridium chloride was added to precipitate hyaluronic acid. After centrifugation, the precipitate was dissolved in 4M NaCl, reprecipitated with ethanol, and this precipitate was dissolved in purified water. For quantitative analysis of hyaluronic acid, electrophoresis was conducted by using cellulose acetate strip [Hata, R. & Nagai, Y. (1972): Anal. Biochem. 45, pp 462-468].
After the electrophoresis, it was soaked in 3% acetic acid containing 0.5% Alcian blue, washed, dried. Then the amount of hyaluronic acid was measured by 600 nm scanner. The result is shown in Table 5.

**Table 5**

| Concentration | Hyaluronic acid synthesis increase effect (%) |
|---|---|
| 100 μg/ml | 35.8 |
| 500 μg/ml | 56.4 |

From the above result, it can be seen that synthesis of hyaluronic acid in normal fibroblast increases as the concentration of kidney bean extracts increases. Therefore, it could be seen that kidney bean extracts exhibits effect of increasing hyaluronic acid synthesis.
The followings are formulation examples of cosmetic composition containing kidney bean extracts of the present invention. They were prepared by the conventional methods in cosmetics field.

**Formulation 1: skin softener**

Skin softener containing kidney bean extracts was prepared by using ingredients and amount described in the following Table 6.

**Table 6**

| Ingredient | Content (% by weight) |
| --- | --- |
| Kidney bean extract | 5.0 |
| 1,3-butylene glycol | 6.0 |
| Sodium hyaluronate | 2.0 |
| Glycerin | 4.0 |
| PEG 4000 | 1.0 |
| Polysorbate 20 | 0.5 |
| Ethanol | 10.0 |
| Preservative | q.s. |
| Benzophenone-9 | 0.1 |
| Flavour | Aequate amount |
| Purified water | q.s. |
| Total | 100 |

**Formulation 2: Milk lotion**

Milk lotion containing kidney bean extracts was prepared by using the ingredient and amount listed in the following Table 7.

**Table 7**

| Ingredient | Content (% by weight) |
| --- | --- |
| Kidney bean extract | 5.0 |
| Stearic acid | 0.4 |
| 1,3-butylene glycol | 6.0 |
| Ccetostearyl alcohol | 1.2 |
| Glycerin | 4.0 |
| Glyceryl stearate | 1.0 |
| Triethanol amine | 0.3 |
| Tocopheryl acetate | 3.0 |
| Liquid Paraffin | 5.0 |
| Squalene | 3.0 |
| Macadamia nut oil | 2.0 |
| Polysorbate 60 | 1.5 |
| Sorbitan sesquioleate | 0.6 |
| Carboxyvinyl polymer | 0.15 |
| Preservative | q.s. |
| Flavour | q.s. |
| Purified water | q.s. |
| Total | 100 |

**Formulation 3: nutrition cream**

Nutrition cream containing kidney bean extracts was prepared by using the ingredient and amount listed in the following Table 8.

**Table 8**

| Ingredient | Content (% by weight) |
|---|---|
| Kidney bean extract | 5.0 |
| Vaseline ® | 7.0 |
| Cetostraryl alcohol | 2.5 |
| Glyceryl stearate | 2.0 |
| Stearic acid | 1.5 |
| Liquid paraffin | 10.0 |
| Bess wax | 2.0 |
| Polysorbate 60 | 1.5 |
| Sorbitan cesquioleate | 0.8 |
| Squalane | 3.0 |
| 1,3-butylene glycol | 6.0 |
| Glycerine | 4.0 |
| Triethanolamine | 0.5 |
| Tocoperyl acetate | 0.1 |
| Preservative | q.s. |
| Flavour | q.s. |
| Purified water | q.s. |
| Total | 100 |

**Formulation 4: Essence**

Essence containing kidney bean extracts was prepared by using the ingredients and amount described in the following Table 9.

**Table 9**

| Ingredient | Content (% by weight) |
|---|---|
| Kidney bean extracts | 5.0 |
| Glycerin | 10.0 |
| PEG 1500 | 2.0 |
| Alantoin | 0.1 |
| Panthenol | 0.3 |
| EDTA | 0.02 |
| Benzophenone-9 | 0.04 |
| Hydroxyethyl cellulose | 0.01 |
| Sodium hyaluronate | 8.0 |
| Carboxyvinyl polymer | 0.2 |

Table continued

| Ingredient | Content (% by weight) |
| --- | --- |
| Triethanolamine | 0.18 |
| Octyldodeces-25 | 0.6 |
| Ethanol | 6.0 |
| Preservative, Flavour, Colouring agent | Very small amount |
| Purified water | q.s. |
| Total | 100 |

## Formulation 5: Massage cream

Massage cream containing kidney bean extracts was prepared by using ingredients and amount described in the following Table 10.

**Table 10**

| Ingredient | Content (% by weight) |
| --- | --- |
| Kidney bean extracts | 3.0 |
| Glyceryl stearate | 2.0 |
| Cetostearyl alcohol | 2.5 |
| Stearic acid | 1.0 |
| Polysorbate 60 | 1.5 |
| Sorbitan stearate | 0.6 |
| Isostearyl isostearate | 5.0 |
| Squalene | 5.0 |
| Mineral oil | 35.0 |
| Dimethicon | 1.0 |
| Xantan gum | 0.1 |
| Hydroxyethyl cellulose | 0.12 |
| Glycerin | 6.0 |
| Triethanolamine | 0.5 |
| Preservative, Flavour, Colouring agent | q.s. |
| Purified water | q.s. |
| Total | 100 |

## Formulation 6: Pack

Pack containing kidney bean extracts was prepared by using the ingredients and amount given in the following Table 11.

**Table 11**

| Ingredient | Content (% by weight) |
| --- | --- |
| Kidney bean extracts | 3.0 |
| Polyvinyl alcohol | 15.0 |
| Cellulose gum | 0.15 |

**EP 1 093 786 B1**

Table continued

| Ingredient | Content (% by weight) |
|---|---|
| Glycerin | 3.0 |
| PEG 1500 | 2.0 |
| Panthenol | 0.4 |
| Alantoin | 0.1 |
| Ethanol | 6.0 |
| PEG 40 hydrogenated castor oil | 0.3 |
| Preservative, flavour, colouring agent | Very small amount |
| Purified water | q.s. |
| Total | 100 |

## Claims

1. Use of a kidney bean extract in a skin cosmetic composition for improving skin wrinkles.

2. Use according to claim 1, wherein the kidney bean extracts are obtained by extracting kidney bean powder with a solvent selected from water, lower alcohol, a mixture of water and lower alcohol, acetone, ethylacetate, 1,3-butylene glycol, hexane, diethyl ether, n-propanol, isopropanol and n-butanol.

3. Use according to claim 1, wherein the kidney bean extracts is contained in an amount of 0.05 to 10.0% based on total dried weight of the cosmetic composition.

4. Use according to any one of claims 1 to 3, where the composition is in the form of basic cosmetics, colour cosmetics or hair cosmetics.

## Patentansprüche

1. Verwendung von Kidney-Bohnen-Extrakt in einer hautkosmetischen Mischung zur Verringerung von Hautfalten.

2. Verwendung nach Anspruch 1, **dadurch gekennzeichnet, dass** der Kidney-Bohnen-Extrakt durch die Extraktion von Kidney-Bohnen-Puder mit einer Lösung, die aus Wasser, niederen Alkoholen und einer Mixtur von Wasser und niederen Alkoholen, Aceton, Ethylacetat, 1,3-Hutylenglycol, Hexan, Diethylether, n-Propanol, Isopropanol und n-Butanol besteht, gewonnen wird.

3. Verwendung nach Anspruch 1, **dadurch gekennzeichnet, dass** der Kidney-Bohnen-Extrakt in einem Anteil von 0,05 bis 10 % des absoluten Trockengewichtes der kosmetischen Mischung enthalten ist.

4. Verwendung nach einem der Ansprüche 1 bis 3, **dadurch gekennzeichnet, dass** die Mischung im Wesentlichen als Grundlage für Kosmetiker, Färbekosmetiker oder Haarkosmetiker Anwendung findet.

## Revendications

1. Application d'un extrait de haricot nain à une composition cosmétique pour la peau destinée à améliorer les rides de la peau.

2. Application selon la revendication 1, dans laquelle les extraits de haricot nain sont obtenus par extraction de poudre de haricot nain avec un solvant choisi parmi l'eau, un alcool inférieur, un mélange d'eau et d'un alcool inférieur, l'acétone, l'acétate d'éthyle, le 1,3-butylèneglycol, l'hexane, l'éther diéthylique, le n-propanol, l'isopropanol et le n-butanol.

3. Application selon la revendication 1, dans laquelle les extraits de haricot nain sont contenus en quantité comprise entre 0,05 et 10,0 % par rapport au poids total à sec de la composition cosmétique;

4. Application selon l'une quelconque des revendications 1 à 3, dans laquelle la composition est sous forme de cosmétique basique, de cosmétique coloré ou de cosmétique pour cheveux.